# DEMANDE DE BREVET EUROPEEN

(11) **EP 0 875 748 A1**
(43) Date de publication de la demande: **04.11.1998**
(21) Numéro de dépôt: 98400771.6
(22) Date de dépôt: 01.04.1998
(51) Int. Cl.: G01N 15/08, G01N 33/24

(54) **Dispositif à membranes semi-perméables, pour tester des échantillons géologiques**

(30) Priorité: 29.04.1997 FR 9705442
(71) Demandeur: Institut Français du Pétrole, 92852 Rueil-Malmaison Cedex (FR)
(72) Inventeur: Fleury, Marc, 78170 La Celle Saint Cloud (FR)

(57) **Abrégé**

- Dispositif pour faire des mesures sur un barreau de roche poreuse en présence de fluides.
- Le dispositif comporte une cellule de confinement comprenant un corps (2) allongé fermé à ses extrémités opposées par des embouts (6, 7) et des moyens de déplacement sous pression à l'intérieur de l'échantillon d'un premier fluide mouillant et d'un deuxième fluide. Contre les extrémités de l'échantillon, on applique une ou plusieurs membranes rigides sélectivement mouillables constituées chacune d'une couche relativement fine d'une céramique poreuse sur une couche support en un matériau poreux. Elles peuvent être réalisées par exemple par frittage de grains métalliques, d'inégales grosseurs pour l'une et pour l'autre (les grains sont beaucoup plus gros pour la couche-support). De telles membranes résistent bien à des températures élevées de l'ordre de plusieurs centaines de °C. On utilise des électrodes (E1) reliées à un appareil de mesure (24) pour déterminer des caractéristiques physiques du barreau.
- Application à l'étude de gisements pétrolifères, notamment.

## Description

La présente demande concerne un dispositif pour faire des mesures sur un échantillon poreux en présence de fluides utilisant des membranes semi-perméables résistant à des températures élevées, dans le but de déterminer certaines de leurs caractéristiques physiques.

Une telle cellule convient par exemple pour tester des échantillons géologiques et déterminer différents paramètres tels que la pression capillaire des roches dans des phases de drainage ou d'imbibition, leurs indices de mouillabilité, leurs perméabilités relatives, leurs indices de résistivité etc. Une telle cellule trouve des applications notamment dans le domaine pétrolier pour tester des roches-réservoirs quand on doit procéder par exemple à une récupération assistée d'un gisement.

Pour déterminer la mouillabilité des roches vis à vis de l'eau et de l'huile qui peuvent y être contenues, on procède par exemple à un drainage de la roche initialement saturée en eau, pour réduire sa saturation (Sw), suivi d'une imbibition, permettant d'augmenter de nouveau sa saturation en eau. La pression capillaire en un point se définit comme la didférence Pc à l'équilibre entre la pression Po de l'huile et celle Pw de l'eau. Ce paramètre n'a de sens que si les deux fluides sont en phase continue dans le milieu poreux. Pour un milieu mouillable à l'eau, seules les valeurs positives ont un sens. Par contre, lorsque le milieu a une mouillabilité mixte, les fluides peuvent rester en phase continue aussi bien pour les pressions capillaires (Pc) positives que négatives.

Pour une application de ce type, un cycle complet de mesure de la pression capillaire doit donc comporter (Fig.4) :
a) un drainage primaire positif d'un échantillon saturé initialement en eau à 100% (courbe 1);
b) une imbibition positive (courbe 2);
c) une imbibition négative (courbe 3);
d) un drainage négatif (courbe 4); et
e) un drainage secondaire positif (courbe 5).

Par la demande de brevet EP-A-0.701.120 du demandeur, on connaît un dispositif pour réaliser des mesures de mouillabilité sur des échantillons de roche poreuse dans une cellule de confinement délimitée à ses deux extrémités opposées par deux embouts. Des canaux traversent ces embouts et mettent leurs faces intérieures rainurées en communication respectivement avec deux sources de fluide à pression ajustable délivrant respectivement un premier et un deuxième fluide. Le barreau est placé dans la cellule à l'intérieur d'une gaine déformable allongée associée à des moyens de pression pour exercer latéralement sur lui une pression de confinement. Entre les faces intérieures des embouts et l'échantillon, sont disposées deux membranes semi-perméables, respectivement perméables au premier et au deuxième fluide. Des électrodes enveloppantes sont placées à l'intérieur de la gaine au contact de la paroi latérale de l'échantillon, de part et d'autre de lui. Elles sont reliées à un ensemble de mesure de la conductivité électrique de l'échantillon. Dans un premier temps, Le barreau est initialement saturé avec un premier fluide tel que de la saumure, avant d'être placé dans la cellule. Un deuxième fluide, de l'huile par exemple, est injecté sous pression à une extrémité de la cellule. Il déplace le premier fluide à l'intérieur de l'échantillon et l'on recueille le volume expulsé à l'extrémité opposée. On procède ensuite à une ou plusieurs des phases suivantes du cycle de mesure rappelées ci-dessus.

Un appareillage de mesure comportant des moyens de mesure de la conductivité électrique ainsi que du volume de fluides expulsé, sont disposés dans la cellule, permettant de déterminer différents paramètres physiques du barreau.

Dans une structure de ce genre, il est connu d'utiliser comme membrane mouillable à l'huile, une membrane par exemple en Goretex (marque déposée) qui supporte une gamme de température (jusqu'à 100 à 200°C) que l'on observe durant les essais d'échantillon poreux.

Comme membrane mouillable à l'eau, on utilise généralement une plaque mince en céramique poreuse qui remplit son rôle tant que la température de fonctionnement de la cellule n'est pas trop grande (de préférence inférieure à 100°C). Il est possible d'utiliser une membrane en céramique de quelques mm d'épaisseur, qui résiste mieux en température mais une telle membrane a pour inconvénient de ralentir notablement le déroulement du cycle d'opérations.

La présente invention concerne un dispositif pour faire des mesures, en présence de fluides, sur un échantillon poreux mouillable par au moins un premier fluide et confiné latéralement par une gaine déformable, dans le but de déterminer des caractéristiques physiques de l'échantillon, qui permet de remédier aux inconvénients ci-dessus énoncés.

Il comporte une cellule comprenant un corps allongé fermé à ses extrémités opposées par des embouts, des moyens de pression pour exercer une pression radiale de confinement sur l'échantillon gainé, des moyens de pompage pour communiquer avec l'intérieur de la cellule au travers des embouts, pour déplacer le premier fluide mouillant et au moins un deuxième fluide, et des moyens de mesure.

Le dispositif est caractérisé en ce qu'il comporte une membrane bi-couche perméable au premier fluide constituée d'un disque de support en un matériau poreux recouvert d'une couche relativement fine d'une céramique poreuse (réalisée par exemple par frittage de grains métalliques) cette membrane bi-couche étant disposée dans la cellule au contact d'une première extrémité de l'échantillon.

Suivant un mode préféré de réalisation, le disque de support est constitué d'un fritté métallique réalisé avec des grains plus gros que ceux utilisés pour constituer la couche fine.

On réalise par exemple une membrane comprenant une couche fine d'un fritté d'alumine avec des grains métalliques de diamètre 0,1 µm, le disque de support étant réalisé à partir d'un fritté d'alumine avec des grains de l'ordre de 100 fois plus gros que ceux utilisé pour la couche fine.

De préférence, la membrane bi-couche est pourvu d'un revêtement périphérique étanche réalisé par exemple en céramique non poreuse, de manière à éviter les traversées de la membrane par contournement.

Le dispositif peut comporter aussi une autre membrane mouillable par le deuxième fluide, disposée au contact de l'extrémité opposée de l'échantillon, qui peut être réalisé en appliquant à une membrane bi-couche analogue à la précédente, un traitement chimique la rendant mouillable par le deuxième fluide.

Avec une telle membrane bi-couche mouillable à un premier fluide tel que l'eau ou la saumure, et éventuellement une autre membrane du même type rendue mouillable à un autre fluide, capables de supporter des températures relativement élevées, on peut réaliser par exemple un dispositif d'étude notamment pour des échantillons géologiques dans des conditions de température et de pression reproduisant celles que l'on peut trouver in situ dans un gisement par exemple, sans être gênés dans le déroulement des cycles de mesure par les limitations propres aux membranes classiquement utilisées.

D'autres caractéristiques et avantages du dispositif selon l'invention, apparaîtront à la lecture de la description ci-après d'un exemple non limitatif de réalisation, en se référant aux dessins annexés où :
- la Fig.1 montre schématiquement en coupe longitudinale un mode de réalisation du dispositif;
- la Fig.2 montre schématiquement un exemple de membrane bi-couche mouillable à l'eau.
- la Fig.3 montre une vue en coupe transversale de ce dernier mode de réalisation; et
- la Fig.4 montre à titre indicatif les variations que subit la pression capillaire dans un échantillon au cours d'un cycle complet de drainage-imbibition;

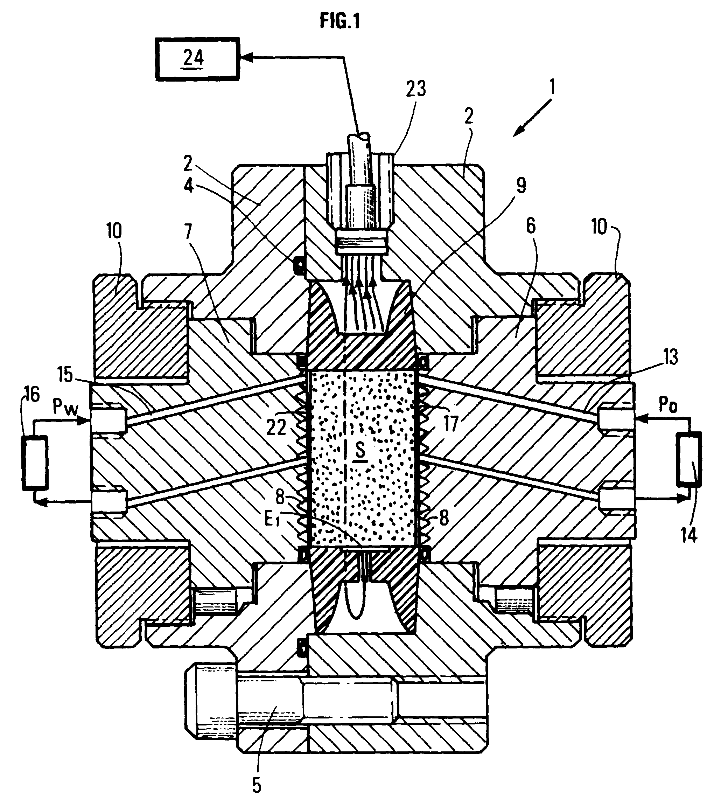

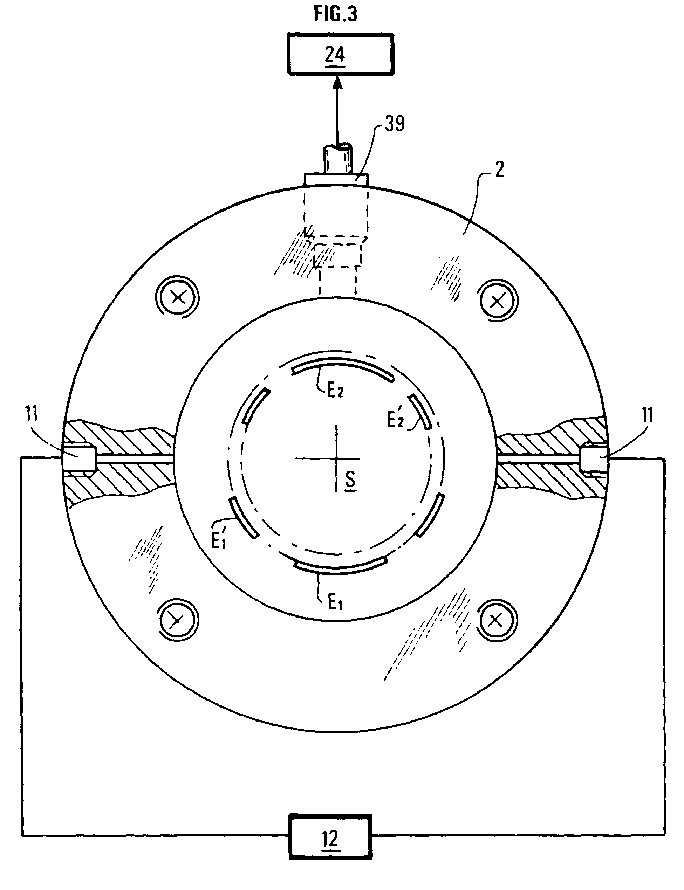

Suivant le mode de réalisation de la Fig.1, le dispositif comporte un coprs creux 1 qui est constitué de deux manchons 2, 3 à symétrie cylindrique. Ils sont appliqués l'un contre l'autre par l'intermédiaire de joints d'étanchéité 4 et réunis par des vis 5. Les deux manchons comportent chacun une cavité axiale pour un embout 6, 7 dont les faces en regard l'une de l'autre sont pourvues de rainures 8 en araignée. L'échantillon S est placé à l'intérieur d'une pièce annulaire en élastomère formant gaine 9. L'ensemble de l'échantillon S et de la gaine 9 est installé dans une cavité intérieure du manchon 3. Les deux embouts 6, 7 sont plaqués contre l'échantillon par vissage de deux écrous 10 dans les deux manchons 2, 3. Deux orifices radiaux 11 (Fig.3) au travers de la paroi extérieure du manchon 2 permettent l'application d'une pression de confinement axiale par une seconde source de pression 12.

Des canaux 13 traversent l'embout 6 et font communiquer le réseau de rainures 8 sur sa face terminale, avec une première source 14 délivrant le premier fluide sous pression. De même, des canaux 15 traversent l'embout 7 et font communiquer le réseau de rainures 8 correspondant avec une deuxième source 16 délivrant le deuxième fluide sous pression.

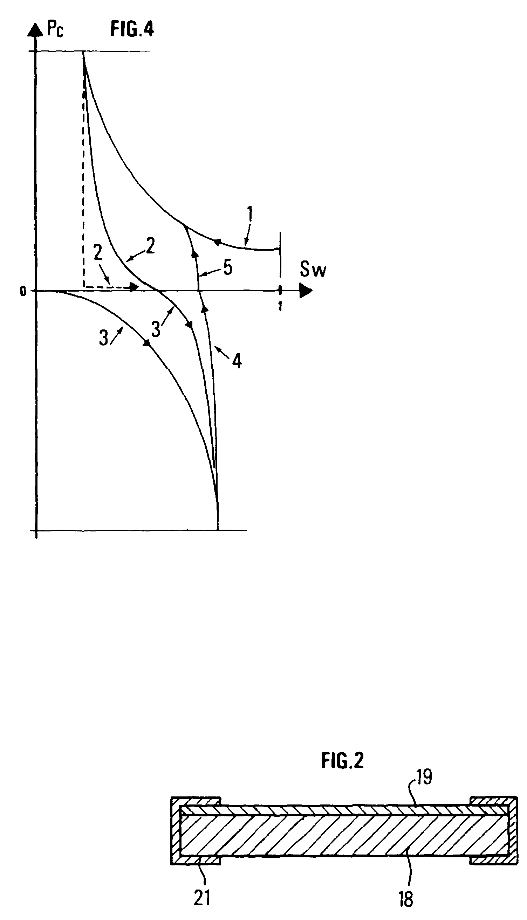

Contre la face de l'un des embouts, est appliquée une première membrane semi-perméable 17 perméable à un premier fluide (de la saumure telle qu'on en trouve dans les échantillons géologiques par exemple). Cette membrane comporte (Fig.2) une première couche 18 perméable à l'eau servant de support à une mince couche fine 19 d'une céramique poreuse. On l'obtient par exemple par frittage de grains métalliques. On peut utiliser notamment un fritté d'alumine.

Une couche fine de 50 µm d'épaisseur, réalisée par frittage à partir de grains métalliques de 0,1 µm de diamètre convient par exemple pour des pressions de l'ordre de quelques dixièmes de Mpa.

Comme couche de support 18, on peut également utilisé un fritté métallique réalisé à partir de grains métalliques plus gros. Typiquement, on utilise une couche de plusieurs millimètres (7mm par exemple) réalisé par frittage de grains de plusieurs µm de diamètre (10 µm par exemple).

Une telle membrane bi-couche en fritté présente l'avantage de très bien résister aux températures relativement élevées reproduisant celles (de l'ordre de plusieurs centaines de °C) auxquelles sont soumises les roches dans les gisements souterrains

Pour éviter les fuites de fluide par contournement, on forme un revêtement annulaire étanche 21 autour de la membrane, débordant sur les deux faces opposées. Ce revêtement est par exemple en céramique non poreuse (émaillage).

Contre la face de l'autre est appliquée une autre membrane semi-perméable 22, perméable à un deuxième fluide tel que de l'huile. On peut utiliser une cloison micro-poreuse d'un type connu fabriqués par exemple par les firmes Gore Tex, Sartorius, Poretics, Millipore etc.

Suivant un mode de réalisation avantageux, la membrane 22 mouillable par le deuxième fluide peut être également une membrane bi-couche analogue à celle décrite ci-dessus, à laquelle on aura appliqué au préalable un traitement chimique approprié. Par exemple, un traitement utilisant une réaction de silanisation (à base de solutions d'organochlorosilanes), permet de rendre la céramique poreuse très fortement mouillable à l'huile. Il est possible aussi d'utiliser la substance appelée Quilon (marque déposée) qui est un complexe de chrome possédant un groupe acide hydrophobe, pour modifier la mouillabilité.

Le dispositif comporte par exemple au moins un premier couple d'électrodes E1, E2 (Fig.3) disposées à l'intérieur de la pièce annulaire 9, permettant l'application d'un courant électrique, et au moins un deuxième couple d'électrodes E'1, E'2 entre lesquelles on mesure la différence de potentiel créée en réponse à l'application du courant électrique. Cette affectation séparée des couples d'électrodes, l'un à l'application d'un courant, et l'autre, à la mesure de différences de potentiel, permet d'éviter les résistances dues aux contacts.

Au travers d'un bouchon 23 pourvu d'une traversée étanche, les fils connectés aux différentes électrodes, sont connectées à un système de mesure de conductivité électrique 24 d'un type connu.

Le dispositif peut être placé dans une enceinte thermorégulée (non représentée) destiner à restituer la température de la formation géologique d'où l'échantillon a été extrait.

## Revendications

1. Dispositif pour faire des mesures en présence de liquides, sur un échantillon poreux mouillable par au moins un premier liquide et confiné latéralement par une gaine déformable (9) dans le but de déterminer des caractéristiques physiques de l'échantillon, comportant une cellule comprenant un corps allongé (2) fermé à ses extrémités opposées par des embouts (6, 7), des moyens de pressions (12) pour exercer une pression radiale de confinement sur l'échantillon gainé, des moyens de pompage (14, 16) communiquant avec l'intérieur de la cellule au travers des dits (6, 7), pour déplacer le premier liquide mouillant et au moins un deuxième liquide, et des moyens de mesure, caractérisé en ce qu'il comporte une membrane bi-couche (17) mouillable par le dit premier liquide, constituée d'un disque de support (18) en un matériau poreux fritté recouvert d'une couche (19) relativement fine en un matériau poreux fritté dont les grains sont beaucoup plus fins de façon que la perméabilité de la membrane bi-couche (17) soit sensiblement celle de la couche fine, cette membrane bi-couche (17) étant disposée dans la cellule au contact d'une première extrémité de l'échantillon (S).

2. Dispositif selon la revendication 1, caractérisé en ce que le rapport entre la taille des grains utilisés pour constituer la couche-support et celle des grains utilisés pour constituer la couche mince, est au moins de 50.

3. Dispositif selon la revendication 2, caractérisé en ce que le dit rapport de taille est choisi égal à 100.

4. Dispositif selon l'une des revendications précédentes, caractérisé en ce que le disque de support (18) et la couche fine (19) sont réalisés par frittage de grains métalliques.

5. Dispositif selon l'une des revendications précédentes, caractérisé en ce que la dite membrane bi-couche (17) perméable au premier liquide comprend une couche fine (19) réalisée à partir d'un fritté d'alumine avec des grains ayant un diamètre de l'ordre de 0,1 mm, le disque de support (18) étant réalisé à partir d'un fritté d'alumine avec des grains de l'ordre de 100 fois plus gros.

6. Dispositif selon l'une des revendications précédentes, caractérisé en ce que la membrane bi-couche (17) est pourvue d'un revêtement périphérique étanche (21), de manière à éviter le franchissement de la membrane par contournement.

7. Dispositif selon l'une des revendications précédentes, caractérisé en ce que le revêtement périphérique étanche est réalisé en céramique non poreuse.

8. Dispositif selon l'une des revendications précédentes, caractérisé en ce qu'il comporte une deuxième membrane bi-couche (22) mouillable par le deuxième liquide, disposée au contact de l'extrémité de l'échantillon (S) opposée à ladite première extrémité.

9. Dispositif selon la revendication précédente, caractérisé en ce que la dite deuxième membrane bi-couche (22) comporte aussi une superposition de deux couches réalisées par frittage de poudre métallique, auxquelles on a appliqué un traitement chimique pour la rendre mouillable par ledit deuxième liquide.

10. Dispositif selon l'une des revendications précédentes, caractérisé en ce qu'il comporte des électrodes (E) disposées autour de l'échantillon et reliées à un appareil (24) de mesure de la résistivité de l'échantillon.
